(19) Europäisches Patentamt
European Patent Office
Office européen des brevets

(11) Numéro de publication: **0 067 777**
**B1**

# (12) FASCICULE DE BREVET EUROPEEN

(45) Date de publication du fascicule du brevet: **10.04.85**

(51) Int. Cl.[4]: **C 07 C 53/12**, C 07 C 51/56

(21) Numéro de dépôt: **82420038.0**

(22) Date de dépôt: **23.03.82**

## (54) Procédé de carbonylation de l'acétate de méthyle.

(30) Priorité: **12.06.81 FR 8111787**

(43) Date de publication de la demande: **22.12.82 Bulletin 82/51**

(45) Mention de la délivrance du brevet: **10.04.85 Bulletin 85/15**

(84) Etats contractants désignés: **AT BE CH DE FR GB IT LI LU NL SE**

(56) Documents cités:
**EP - A - 0 008 396**
**EP - A - 0 031 784**
**FR - A - 2 242 362**
**US - A - 4 115 444**

(73) Titulaire: **RHONE-POULENC CHIMIE DE BASE, 25, quai Paul Doumer, F-92408 Courbevoie (FR)**

(72) Inventeur: **Gauthier-Lafaye, Jean, 21, rue Duguesclin, F-69006 Lyon (FR)**
Inventeur: **Perron, Robert, La Pécolière, F-69390 Charly (FR)**

(74) Mandataire: **Varnière-Grange, Monique et al, RHONE-POULENC RECHERCHES Service Brevets Chimie et Polymères Centre de Recherches de Saint-Fons 85, rue des Frères Perret B.P. 62, F-69192 St-Fons Cédex (FR)**

EP 0 067 777 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

**Description**

La présente invention a pour objet un procédé de carbonylation de l'acétate de méthyle dans un milieu pratiquement anhydre.

La présente invention a plus particulièrement pour objet un perfectionnement au procédé de carbonylation de l'acétate de méthyle dans un milieu pratiquement anhydre, mettant en œuvre un catalyseur à base de cobalt.

Le brevet américain n° 2 730 546 décrit la carbonylation de l'acétate de méthyle en anhydride acétique en présence d'un catalyseur choisi parmi les complexes du cobalt de formule générale:

$$[R_4A]_2\ CoX_4$$

dans laquelle X représente un atome de brome ou d'iode, A un atome d'azote ou de phosphore et R représente un radical alkyle inférieur, par exemple.

Ces complexes peuvent être formés in-situ en chargeant d'une part un halogénure du cobalt ($CoX'_2$) et d'autre part un halogénure d'ammonium (ou de phosphonium) quaternaire ($R_4AX$). La formation des complexes en question peut alors être représentée par la réaction suivante:

$$2(R_4AX) + CoX'_2 \rightarrow [R_4A]_2\ CoX'_2X_2$$

Toutefois, l'efficacité de ces catalyseurs à base de cobalt paraît relativement faible. Ce type de procédé, dont l'intérêt de principe n'est pas contesté, n'a pu déboucher sur une réalisation industrielle.

Le brevet français n° 2 242 362 (correspondant aux demandes américaines n° 394 220 et n° 467 977, respectivement du 4 septembre 1973 et du 8 mai 1974) décrit un procédé de préparation de l'anhydride acétique en deux stades, dans lequel, lors d'une première étape, le bromure, ou de préférence l'iodure, de méthyle est carbonylé pour obtenir l'halogénure d'acétyle correspondant, cet halogénure d'acétyle étant à son tour mis à réagir sur de l'acétate de méthyle pour conduire, dans une deuxième étape, à l'anhydride acétique, ce qui correspond au schéma réactionnel suivant, dans le cas où l'iodure de méthyle est la matière de départ:

— *Etape 1:*

$$CH_3I + CO \rightarrow CH_3COI$$

— *Etape 2:*

$$CH_3COI + CH_3COOCH_3 \rightarrow (CH_3CO)_2O + CH_3I$$

Comme ce schéma le fait apparaître clairement, l'iodure de méthyle, matière de départ de l'étape 1, est «régénéré» dans l'étape 2. L'étape 1 est avantageusement conduite en présence de catalyseur à base de rhodium; l'étape 2 serait favorisée par la présence de lithium et/ou de chrome. Les deux étapes peuvent être réalisées dans une même zone réactionnelle qui renfermera alors de l'iodure de méthyle, de l'acétate de méthyle, du rhodium et le cas échéant de lithium et/ou du chrome, voire de l'iodure d'acétyle, zone dans laquelle on introduira également du monoxyde de carbone.

Le brevet américain n° 4 115 444 propose un perfectionnement de la technique décrite dans le brevet français précité, perfectionnement qui consiste à ajouter au milieu réactionnel un composé organique du phosphore ou de l'azote, dans lequel le phosphore ou l'azote est trivalent et confirme l'intérêt potentiel des systèmes catalytiques à base de rhodium, voire de palladium ou d'iridium, et de chrome dans cette réaction.

Le brevet français n° 2 303 788 (correspondant aux demandes américaines n° 556 750 et n° 654 661 respectivement du 10 mars 1975 et du 5 février 1976) montre que la présence d'une quantité importante d'hydrogène dans le milieu réactionnel défini ci-avant influe de manière notable sur l'orientation de la réaction. En effet on obtient dans ces conditions un mélange renfermant une proportion prépondérante d'acide acétique, des quantités variables de diacétate d'éthylidène, d'anhydride acétique et d'acétaldéhyde.

L'intérêt principal de ces procédés mettant en œuvre des catalyseurs à base de rhodium, voire de palladium ou d'iridium, les systèmes basés sur le couple (rhodium-chrome) paraissant être les plus actifs, réside essentiellement dans la possibilité qu'ils offrent, d'accéder à l'anhydride acétique au départ d'acétate de méthyle en utilisant des pressions partielles de monoxyde de carbone plus basses que celles requises dans les procédés antérieurs.

Néanmoins, les tentatives de développement de ce type de procédés, même sur une simple échelle pré-industrielle, se heurtent à des difficultés non-négligeables.

Une première série de difficultés réside dans le fait que les catalyseurs à base de rhodium ou de palladium, voire d'iridium, métaux extrêment rares et coûteux, se désactivent notamment lors des traitements nécessaires à la récuperation du (ou des) produit(s) de la réaction. En raison du coût de ces catalyseurs il est indispensable de les régénérer. Par ailleurs les conditions nécessaires pous transformer ces composés métalliques en espèces catalytiquement actives dans la réaction de carbonylation sont le plus souvent incompatibles avec celles nécessaires au maintient des cocatalyseurs, à base de chrome, sous leur forme active dans cette même réaction. Par ailleurs les pertes en rhodium, par exemple, qui paraissent inévitables dans les divers points d'une unité indus-trielle grèvent lourdement l'économie d'un tel procédé.

Une seconde série de difficultés trouve sa source dans la présence nécessaire au bon déroulement de la réaction et à la stabilisation du rhodium, de grandes quantités d'iodure de méthyle (ou d'acétyle) qui implique des risques importants de corrosion dans les divers points d'une installation industrielle. Par ailleurs l'iodure de méthyle et/ou certains de ses dérivés formés dans le milieu réactionnel sont responsables d'une contamination intolérable du (ou des) produit(s) réactionnel(s) qui nécessite la mise en œuvre d'étapes supplémentaires pour éliminer les iodures dont la présence s'avère indésirable dans les produits de réaction. Ces dérivés iodés présents en grandes quantités non seulement dans les produits mais aussi dans divers effluents issus de la zone réactionnelle doivent, pour des raisons économiques évidentes,

être récupérés ce qui implique des stades de traitement additionnels.

Les divers problèmes, délicats à résoudre, associés à ce type de procédés apparaîtront plus clairement à la lecture des brevets français n° 2 438 023 et n° 2 438 024 (correspondant respectivement aux demandes américaines n° 949 344 et n° 949 345 déposées le 6 octobre 1978) et du brevet américain n° 4 241 219.

Par ailleurs, il est bien connu que l'acétate de méthyle peut être obtenu par réaction de l'acide acétique et du méthanol, l'acide acétique pouvant être produit par carbonylation du méthanol et le méthanol pouvant être à son tour fabriqué par hydrogénation du monoxyde de carbone. Les reactions en cause sont susceptibles d'être représentées comme suit:

$CO + 2H_2 \rightarrow CH_3OH$ (a)
$CH_3OH + CO \rightarrow CH_3COOH$ (b)
$CH_3COOH + CH_3OH \rightarrow CH_3COOCH_3 + H_2O$ (c)

La carbonylation de l'acétate de méthyle en milieu sensiblement anhydre permet d'obtenir l'anhydride acétique selon la réaction suivante:

$CH_3COOCH_3 + CO \rightarrow CH_3CO\text{-}O\text{-}COCH_3$ (1)

Ainsi, l'intérêt d'un procédé de carbonylation de l'acétate de méthyle en anhydride acétique (1) apparaît clairement lorsqu'on considère également les réactions (a) à (c) ci-avant, puisque globalement cet ensemble revient à un procédé par lequel l'anhydride acétique est produit au départ de monoxyde de carbone et d'hydrogène.

D'autre part, le cobalt étant un métal courant, on conçoit aisément l'intérêt potentiel de son utilisation dans un procédé de carbonylation de l'acétate de méthyle. Il est donc étonnant de constater que toutes les solutions antérieures proposées récemment reposent sur la préconisation de systèmes catalytiques à base de rhodium.

Il a maintenant été trouvé de manière tout à fait surprenante qu'il est possible de carbonyler efficacement l'acétate de méthyle, dans un milieu pratiquement anhydre, en faisant appel à un système catalytique à base de cobalt.

La présente invention a donc pour objet un procédé de préparation de l'anhydride acétique le cas échéant en mélange avec l'acétaldéhyde, et/ou le diacétate d'éthylidène par réaction en phase liquide homogène dans un milieu pratiquement anhydre de l'acétate de méthyle et du monoxyde de carbone, caractérisé en ce qu'on opère en présence simultanée:

— (a) d'une source de cobalt,
— (b) d'une source de ruthénium,
— (c) d'un iodure ionique de formule:

$$A^{m+}\ I^{-}m$$

dans laquelle $A^{m+}$ est un cation choisi dans le groupe constitué par les cations onium quaternaire dérivant des éléments du groupe de l'azote et les cations des métaux alcalins, les cations des métaux alcalino-terreux, les cations des métaux du groupe des lanthanides et les cations des métaux du groupe des actinides, m étant un entier égal à 1, 2, 3 ou 4,

— (d) le cas échéant, d'un carboxylate de formule:

$$A'^{n+}\ (OCOR)^{-}_{n}$$

dans laquelle n est égal à 1, 2, 3 ou 4 et $A'^{n+}$ a la signification donnée ci-avant pour $A^{m+}$, $A'^{n+}$ et $A^{m+}$ pouvant par ailleurs être identiques où différents, et R est un radical alkyle, aralkyle ou aryle ayant au maximum 8 atomes de carbone, et

—(e) d'hydrogène, l'hydrogène représentant de 1 à 50% en volume du mélange de monoxyde de carbone et d'hydrogène, la température de réaction étant comprise entre 60 et 300°C, et la pression totale en température étant comprise entre 20 et 300 bars, et la quantité totale de composés halogénés présente dans le milieu réactionnel (exprimée en atome-gramme d'halogène et désignée par $X_T$) étant telle que le rapport atomique $X_T/(m.A^{m+} + n.A'^{n+})$ soit inférieur ou égal à 1.

Le présent procédé de carbonylation de l'acétate de méthyle est remarquable en divers points. En effet il a été trouvé de manière tout à fait inattedue que l'adjonction d'une source de ruthénium et d'hydrogène au milieu réactionnel renfermant des ingrédients du type (a), du type (c) et, le cas échéant, du type (d) mentionnés ci-avant, permet d'obtenir notamment de l'anhydride acétique, avec une productivité horaire particulièrement élevée, alors que dans les mêmes conditions réactionnelles, le cobalt, seul, ne possède qu'une médiocre activité carbonylante dans la réaction envisagée et que le ruthénium, seul, dans ces mêmes conditions, ne développe pratiquement aucune activité carbonylante.

Sans pour autant être lié par une quelconque explication (ou mécanisme réactionnel), on est conduit par ce faits surprenants à penser que l'activité carbonylante doit être attribuée, dans le présent procédé, à la source de cobalt qui, dans les conditions réactionnelles, se transformerait en une espèce catalytiquement active, dont la nature précise n'est pas totalement élucidée.

Le second constituant métallique du système (source de ruthénium), qui ne possède en lui-même pratiquement aucune activité carbonylante, ne servirait apparamment qu'à augmenter l'activité et/ou la concentration en espèce active du cobalt ou sa durée de vie, le cas échéant. La raison de cette influence remarquable est inconnue mais pourrait cependant résider dans un changement du dégré d'oxydation et/ou de la disposition, voire de la nature, des coordinats de l'atome central de cobalt. En d'autres termes, la source de ruthénium aurait pour fonction de faciliter l'apparition et, le cas échéant le maintien, dans le milieu réactionnel de la forme active du cobalt.

Par ailleurs il a été trouvé de manière tout à fait surprenante que, contrairement à l'enseignement de l'art antérieur, l'efficacité du présent procédé n'est pas liée à la présence de grandes quantités d'iodure de méthyle. Au contraire, lorsqu'on ajoute de l'iodure de méthyle lors de la mise en œuvre du présent procédé, on observe une baisse notable de son efficacité.

La raison précise de l'influence négative de l'iodure

de méthyle, phénomène en contradiction totale avec l'enseignement de l'art antérieur, est inconnue. La Demanderesse, sans pour autant être liée par une quelconque explication, suppose que l'iodure de méthyle, lorsqu'il est présent en quantités notables, rompt les équilibres complexes qui seraient notamment à l'origine de la très grande réactivité du cobalt dans les conditions décrites.

Par contre l'activité carbonylante du système précédemment défini n'est pas liée à la nature précise du ou des composés du cobalt chargés initialement. N'importe quelle source de cobalt susceptible de réagir avec l'oxyde de carbone dans le milieu de réaction pour donner des complexes carbonyles du cobalt peut être utilisée dans le cadre du présent procédé. Les sources typiques du cobalt sont par exemple le cobalt métallique finement divisé, des sels inorganiques de cobalt (nitrate, carbonate, halogénures...) ou des sels organiques en particulier des carboxylates. Peuvent également être employés les cobalt-carbonyles ou hydrocarbonyles et les sels de l'hydrotétracarbonylcobalt. Parmi les dérivés du cobalt convenant à la mise en œuvre du procédé selon l'invention, on peut citer le formiate, l'acétate et plus particulièrement le dicobaltoctacarbonyle, le tétracarbonylcobaltate de lithium, le tétracarbonylcobaltate de sodium et le tétracarbonylcobaltate de méthyltriphénylphosphonium.

La quantité précise de cobalt engagée à la réaction n'est pas fondamentale. En général, on opère avec une quantité de cobalt telle que la concentration dans le milieu réactionnel exprimée en milliatomes-grammes par litre (mAt-g/l) soit comprise entre 0,1 et 200 et de préférence entre 0,5 et 100 mAt-g/l.

Un avantage du présent procédé réside dans le fait qu'il est possible d'obtenir de bons résultats avec une faible concentration en cobalt.

Pour mettre en œuvre le présent procédé il est également fait appel à une source de ruthénium. La forme précise sous laquelle le ruthénium est chargé dans le milieu réactionnel initial n'est pas fondamentale. Des sources typiques de ruthénium sont par exemple le ruthénium métallique finement divisé, les carboxylates de ruthénium (notamment l'acétate), l'acétylacétonate de ruthénium, les ruthénium carbonyles [en particulier, $Ru_3(CO)_{12}$].

La quantité de ruthénium présente dans le milieu réactionnel initial n'est pas fondamentale. De manière générale cette quantité sera telle que le rapport atomique Ru/Co soit compris entre 0,005 et 25. Pour une bonne mise en œuvre du présent procédé le rapport atomique Ru/Co sera compris entre 0,02 et 10 et de préférence entre 0,2 et 5.

Le procédé selon la présente invention nécessite également la présence d'un iodure ionique de formule:

$$A^{m+}\ I^{-}m$$

dans laquelle $A^{m+}$ et m ont la signification précédente.

Par cations onium quaternaire dérivés des éléments du groupe de l'azote on entend des cations formés à partir d'azote, de phosphore, d'arsenic ou d'antimoine et de quatre groupements hydrocarbonés monovalents, identiques ou différents, dont la valence libre est portée par un atome de carbone, chaque groupement étant relié à l'élément précité par ladite valence libre, deux quelconques de ces groupements pouvant par ailleurs former ensemble un radical unique divalent.

Parmi ces composés on préconise l'emploi d'iodures de phosphonium (ou d'ammonium) quaternaire. Les cations de ces iodures peuvent être représentés de manière commode par les formules (I) à (III) ci-après:

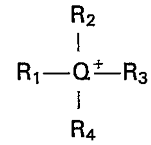

(I)

dans laquelle Q représente un atome d'azote ou de phosphore, $R_1$, $R_2$, $R_3$ et $R_4$, pouvant être identiques ou différents représentent des radicaux organiques dont la valence libre est portée par un atome de carbone, deux quelconques de ces divers radicaux pouvant éventuellement former ensemble un radical unique divalent.

Plus spécifiquement $R_1$, $R_2$, $R_3$ et $R_4$ peuvent représenter des radicaux alkyles linaires ou ramifiés, des radicaux cycloalkyles, aralkyles (par exemple benzyle) ou aryles monocycliques, ayant au plus 16 atomes de carbone, pouvant le cas échéant être substitués par 1 à 3 radicaux alkyles ayant de 1 à 4 atomes de carbone; deux des radicaux $R_1$ à $R_4$ pouvant éventuellement former ensemble un radical unique divalent — alkylène ou alcénylène — comportant 3 à 6 atomes de carbone (par exemple un radical tétraméthylène ou hexaméthylène) et le cas échéant 1 ou 2 doubles liaisons éthyléniques, ledit radical pouvant porter 1 à 3 substituants alkyles ayant de 1 à 4 atomes de carbone.

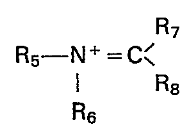

(II)

dans laquelle $R_5$, $R_6$, $R_7$ et $R_8$ identiques ou différents représentent des radicaux alkyles de 1 à 4 atomes de carbone, l'un des radicaux $R_7$ ou $R_8$ pouvant en outre représenter l'hydrogène, $R_7$ et $R_8$ pouvant éventuellement former ensemble un radical unique divalent alkylène comportant de 3 à 6 atomes de carbone, tétraméthylène ou hexaméthylène par exemple; $R_6$ et $R_7$ ou $R_8$ peuvent former ensemble un radical unique divalent — alkylène ou alcénylène — comportant 4 atomes de carbone et le cas échéant 1 ou 2 doubles liaisons éthyléniques, l'atome d'azote étant alors inclus dans un hétérocycle, pour constituer, par exemple, un cation pyridinium.

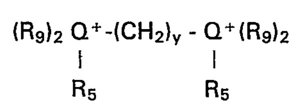

(III)

dans laquelle $R_5$ et $Q^+$ ont la signification donnée précédemment, $R_9$ pouvant être identique à $R_5$ représente un radical alkyle ayant de 1 à 4 atomes de carbone ou un radical phényle, et y est un entier

compris entre 1 et 10 inclus et de préférence entre 1 et 6 inclus. A titre d'exemple d'iodures d'ammonium quaternaire convenant à la mise en œuvre du présent procédé on peut citer les iodures

de tétraméthylammonium,
de triéthylméthylammonium,
de tributylméthylammonium,
de triméthyl(n-propyl)ammonium,
de tétraéthylammonium,
de tétrabutylammonium,
de dodécyltriméthylammonium,
de benzyltriméthylammonium,
de benzyldiméthylpropylammonium,
de benzyldiméthyloctylammonium,
de diméthyldiphénylammonium,
de méthyltriphénylammonium,
de N,N-diméthyl-triméthylénammonium,
de N,N-diéthyl-triméthylénammonium,
de N,N-diméthyl-tétraméthylénammonium,
de N,N-diéthyl-tétraméthylénammonium,
de N-méthylpyridinium,
de N-éthylpyridinium,
de N-méthylpicolinium.

A titre d'exempe d'iodures de phosphonium quaternaire convenant également à la mise en œuvre du présent procédé, on peut citer les iodures

de tétraméthylphosphonium,
d' éthyltriméthylphosphonium,
de triméthylpentylphosphonium,
d' octyltriméthylphosphonium,
de dodécyltriméthylphosphonium,
de triméthylphénylphosphonium,
de diéthyldiméthylphosphonium,
de dicyclohexyldiméthylphosphonium,
de diméthyldiphénylphosphonium,
de cyclohexyltriméthylphosphonium,
de triéthylméthylphosphonium,
de méthyl-tri(isopropyl)phosphonium,
de méthyl-tri(n-propyl)phosphonium,
de méthyl-tri(n-butyl)phosphonium,
de méthyl-tri(méthyl-2 propyl)phosphonium,
de méthyltricyclohexylphosphonium,
de méthyltriphénylphosphonium,
de méthyltribenzylphosphonium,
de méthyl-tri(méthyl-4 phényl)phosphonium,
de méthyltrixylylphosphonium,
de diéthylméthylphénylphosphonium,
de dibenzylméthylphénylphosphonium,
d' éthyltriphénylphosphonium,
de tétraéthylphosphonium,
d' éthyl-tri(n-propyl)phosphonium,
de triéthylpentylphosphonium,
d' éthyltriphénylphosphonium,
de n-butyl-tri(n-propyl)phosphonium,
de butyltriphénylphosphonium,
de benzyltriphénylphosphonium,
de (β-phényléthyl)diméthylphénylphosphonium,
de tétraphénylphosphonium,
de triphényl(méthyl-4 phényl)phosphonium.

La nature précise du cation ammonium ou phosphonium quaternaire n'est pas fondamentale dans le cadre du présent procédé. Le choix parmi ces composés est davantage orienté par des considérations d'ordre pratique, telles que la solubilité dans le milieu réactionnel, la disponibilité et la commodité d'emploi.

A ce titre, les iodures d'ammonium ou de phosphonium quaternaire représentés soit par la formule (I) dans laquelle l'un quelconque des radicaux $R_1$ à $R_4$ est choisi parmi les radicaux alkyles linéaires ayant de 1 à 4 atomes de carbone, soit par les formules (II) ou (III) dans laquelle $R_5$ (ou $R_6$) est également un radical alkyle ayant de 1 à 4 atomes de carbone, conviennent particulièrement bien.

En outre, parmi les iodures d'ammonium on préfère ceux dont les cations correspondent à la formule (I) dans laquelle tous les radicaux $R_1$ à $R_4$ sont choisis parmi les radicaux alkyles linéaires ayant de 1 à 4 atomes de carbone et dont au moins trois d'entre eux sont identiques.

De même, parmi les iodures de phosphonium quaternaire, on préfère ceux dont les cations correspondent à la formule (I) dans laquelle l'un quelconque des radicaux $R_1$ à $R_4$ représente un radical alkyle linéaire ayant de 1 à 4 atomes de carbone, les trois autres radicaux étant identiques et choisis parmi les radicaux phényle, tolyle ou xylyle.

Les iodures des métaux alcalins, des métaux alcalino-terreux, des métaux du groupe des lanthanides et des métaux du groupe des actinides conviennent également à la mise en œuvre de la présente invention. L'emploi de ce type d'iodures doit être plus particulièrement préconisé, lorsqu'on souhaite mettre en œuvre le présent procédé à une température inférieure à 160°C et sous une pression totale en température inférieure à 100 bars, voire inférieure à 80 bars. Les iodures des métaux alcalins, notamment l'iodure de lithium, conviennent plus particulièrement à la mise en œuvre du présent procédé. Toutefois pour avoir un milieu réactionnel homogène dans le cas de tels iodures sont mis en œuvre il convient d'introduire également dans le milieu réactionnel un solvant choisi parmi la tétraméthylènesulfone, la N-méthylpyrrolidone et les amides d'acides monocarboxyliques, qui dérivent d'acides ayant au maximum 8 atomes de carbone, et dont l'atome d'azote comporte deux substituants alkyles ayant au maximum 4 atomes de carbone. Ce type de solvant est utilisé à raison de 5 à 90% (en volume) du milieu réactionnel, bien que des proportions supérieures ou inférieures puissent être mises en œuvre. Pour une bonne mise en œuvre de l'invention le solvant représentera dans le cadre de cette variante de 10 à 60% en volume dudit milieu réactionnel.

La quantité d'iodure ionique à mettre en œuvre dans le cadre du présent procédé est en général telle que le rapport molaire $I^-/Co$ soit supérieur ou égal à 5 et de préférence, supérieur ou égal à 10. Il n'est pas utile que ce rapport dépasse la valeur de 200. On fixera avantageusement le rapport molaire $I^-/Co$ à une valeur comprise entre 15 et 150.

Comme indiqué en tête du présent mémoire, la présente invention peut être mise en œuvre également en présence d'un carboxylate de formule:

$$A'^{n+} (OCOR)^-_n$$

dans laquelle n est égal à 1, 2, 3 ou 4 et $A'^{n+}$ a la signification donnée ci-avant pour $A^{m+}$, $A'^{n+}$ et $A^{m+}$ pouvant par ailleurs être identiques ou différents, et R est un radical alkyle, aralkyle ou aryle ayant au maximum 8 atomes de carbone.

Ce mode d'exéctution constitue une variante avantageuse du présent procédé lorsqu'on fait appel à un iodure alcalin ou alcalino-terreux, ou aux iodures des métaux du groupe des lanthanides ou du groupe des actinides. En effet les carboxylates en cause semblent limiter l'apparition in-situ d'iodure de méthyle (dont on a déjà signalé l'effet néfaste dans le présent procédé) susceptibles de se former par réaction desdits iodures sur l'acétate de méthyle (matière de départ) selon le schéma réactionnel donné ci-après dans le cas particulier de l'iodure de lithium, la formation d'iodure de méthyle étant plus importante au départ d'iodure de lithium qu'au départ d'une quantité équivalente d'iodure de sodium ou de potassium (par exemple):

$$Li^{+}I^{-} + CH_3COOCH_3 \rightarrow CH_3I + Li^{+}(OCOCH_3)^{-}$$

Il n'est pas nécessaire que le carboxylate $A'^{n+}(OCOR)^{-}_{n}$ dérivé du même cation que l'iodure ionique utilisé. Le carboxylate est avantageusement un acétate.

On constatera que certains acétates peuvent être considérés comme le produit d'addition de l'acétate de méthyle et d'une phosphine, d'une amine, d'une arsine ou d'une stibine selon le schéma donné dans le cas particulier de la triphénylphosphine pour simplifier l'exposé: $CH_3COOCH_3 + (C_6H_5)_3P \rightarrow (CH_3)(C_6H_5)_3P^{+}(OCOCH_3)^{-}$.

C'est la raison pour laquelle chaque mole de phosphine, d'amine, d'arsine ou de stibine, éventuellement chargée ou alimentée sera assimilée à un équivalent-gramme de cation $A'^{n+}$ dans la condition reliant la quantité totale d'halogène ($X_T$) à celle des cations $A^{m+}$ et $A'^{n+}$ présente dans le milieu réactionnel.

Lorsqu'on utilise un carboxylate du type défini précédemment sa quantité est en général telle que le rapport molaire $A'^{n+}/A^{m+}$ soit compris entre 0,01 et 20. De préférence, ce rapport sera compris entre 0,05 et 10 de manière avantageuse entre 0,1 et 5.

Enfin, la mise en œuvre du présent procédé nécessite la présence d'hydrogène. L'hydrogène représentant de 1 à 50% en volme du mélange de monoxyde de carbone et l'hydrogène. La quantité maximale d'hydrogène admissible dans le cadre du présent procédé dépendra des autres conditions opératoires et pour une large mesure de la nature du (ou des) produit(s) visé(s). En effet lorsque la quantité d'hydrogène est fixée à une valeur proche de seuil minimal indiqué ci-avant, le procédé selon la présente invention permet d'accéder efficacement et sélectivement à l'anhydride acétique. Par contre en présence de quantités d'hydrogène bien supérieures au seuil dont il vient d'être question, l'efficacité du procédé n'est pratiquement pas modifiée mais on observe des réactions secondaires dont la plus importante peut être représentée comme suit:

$$CH_3COOCH_3 + CO + H_2 \rightarrow CH_3CHO + CH_3COOH \quad (2)$$

ainsi que la réaction latérale suivante:

$$(CH_3CO)_2O + CH_3CHO \rightarrow CH_3CH(OCOCH_3)_2 \quad (3)$$

qui traduit la formation du diacétate d'éthylidène à partir de l'anhydride acétique et de l'acétaldéhyde, ce dernier étant formé par la réaction secondaire (2), ces réactions étant surtout observées à haute température. Les divers produits alors susceptibles de se former en plus de l'anhydride acétique ne sont toutefois pas dépourvus d'intérêt. En effet le diacétate d'éthylidène est un intermédiaire de la synthèse de l'acétate de vinyle; l'acide acétique peut être recyclé dans la préparation de l'acétate de méthyle et l'acétaldéhyde peut être hydrogéné en éthanol.

Par milieu réactionnel pratiquement anhydre, on entend un milieu qui renferme le moins d'eau possible compte-tenu des diverses contraintes industrielles. On notera toutefois, que la présence de quantités mineures d'eau telles que celles susceptibles d'être apportées dans la charge ou l'alimentation par des réactifs de qualité technique est tolérable.

Comme indiqué en tête du présent mémoire il est nécessaire que la quantité totale de composés halogénés présente dans le milieu réactionnel ($X_T$, exprimée en atome-gramme d'halogène) soit telle que le rapport (atomique) $X_T/(m.A^{m+} + n.A'^{n+})$ soit inférieur ou égal à 1.

Cette condition n'exclut pas la possibilité de faire appel à des halogènes ($X_2$), à des acides halogénés (HX), à des halogénures d'alkyle (RX), à des halogénures de cobalt ($CoX_2$) ou à des halogénures de ruthénium ($RuX_3$), mais elle implique que si l'on charge ou alimente ces types de composés on devra nécessairement charger ou alimenter soit un carboxylate $[A'^{n+}(OCOR)^{-}_{n}]$ défini précédemment, soit une phosphine, une amine, une arsine ou une stibine, en quantité au moins équivalente à la quantité d'atomes-grammes d'halogène (X) introduite, le cas échéant, au moyen des composés halogénés mentionnés ci-avant. On admet que dans les conditions réactionnelles, les composés halogénés $X_2$, HX, $CoX_2$ et $RuX_3$ vont réagir sur l'acétate de méthyle pour donner naissance à un halogénure de méthyle, qui réagira à son tour (ainsi que les halogénures d'alkyle RX) sur la phosphine, l'amine, l'arsine ou la stibine pour former l'halogénure d'onium quaternaire correspondant.

C'est la raison pour laquelle, dans l'hypothèse où on fait appel aux composés halogénés de types $X_2$, HX, RX, $CoX_2$ et $RuX_3$, chaque mole de phosphine, d'amine, d'arsine ou de stibine introduite pour «neutraliser» en quelque sorte ces sources d'halogène sera considérée comme un équivalent-gramme de cation $A^{m+}$ dans la condition reliant la quantité totale d'halogène ($X_T$) à celle des cations $A^{m+}$ et $A'^{n+}$ présente dans le milieu réactionnel.

L'halogénure de méthyle $CH_3X$, au même titre que les halogénures d'alkyle (RX) peut par ailleurs réagir sur les carboxylates $[A'^{n+}(OCOR)^{-}_{n}]$ pour donner naissance aux halogénures ioniques correspondants $(A'^{n+}X^{-}_{n})$.

Les phosphines. amines, arsines et stibines auxquelles on recourra, le cas échéant, peuvent être représentées par la formule (IV) ci-après:

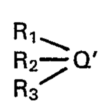
(IV)

dans laquelle Q' représente un atome de phosphore, d'azote, d'arsenic ou d'antimoine, et $R_1$ à $R_3$ ont la même signification que précédemment.

On recourt avantageusement à une phosphine, et plus particulièrement à la triphénylphosphine.

Lorsqu'on choisit de mettre en œuvre le procédé selon l'invention à une température supérieure ou égale à 160°C, voire supérieure à 180°C, il sera avantageux d'opérer sous une pression totale en température supérieure ou égale à 100 bars et il sera préférable que le milieu réactionnel initial renferme outre de l'acétate de méthyle et les divers constituants du système catalytique définis dans le présent mémoire, un solvant choisi parmi les acides carboxyliques aliphatiques ayant au maximum 8 atomes de carbone. Parmi ces acides la Demanderesse préconise l'emploi de l'acide acétique. Pour une bonne mise en œuvre du procédé selon l'invention, l'acide carboxylique représente de 1 à 75% en volume du milieu réactionnel, et encore mieux de 5 à 30% (en volume) dudit milieu.

Le milieu réactionnel initial peut également renfermer dans ce cas de 10 à 50% en volume d'un solvant additionnel choisi dans le groupe constitué par la tétraméthylènesulfone, la N-méthylpyrrolidone et les amides d'acides monocarboxyliques, qui dérivent d'acides ayant au maximum 8 atomes de carbone, et dont l'atome d'azote comporte deux substituants alkyles ayant au maximum 4 atomes de carbone et l'anhydride acétique.

La pression partielle du monoxyde de carbone mesurée à 25°C est en général supérieure à 5 bars et, de préférence supérieure à 10 bars.

La température de réaction qui peut varier dans de larges limites est en général comprise entre 60 et 300°C. On opère, avantageusement, à une température comprise entre 80 et 240°C et, de préférence à une température supérieure à 100°C, gamme dans laquelle le système catalytique développe une efficacité optimale.

Un avantage du présent procédé réside dans la possibilité de carbonyler efficacement l'acétate de méthyle même avec une faible concentration de cobalt, de ruthénium et d'iodure ionique, sous une pression totale en température de l'ordre de 20 à 300 bars, qui est donc bien inférieure à celle habituellement préconisée pour des systèmes catalytiques à base de cobalt.

Un autre avantage du présent procédé réside dans la disparition des diverses contraintes associées à l'utilisation des systèmes catalytiques récemment proposés pour réaliser cette carbonylation.

Comme les spécialistes concernés le savent parfaitement bien, l'acétate de méthyle, matière de départ dans le présent procédé, peut être formé in-situ à partir de diméthyléther, aussi est-il tout à fait possible dans le cadre de la présente invention, de charger ou d'alimenter du diméthyléther ou un mélange de diméthyléther et d'acétate de méthyle.

En fin d'opération, les produits obtenus peuvent être aisément séparés, par distillation fractionnée du mélange résultant, par exemple.

Les exemples ci-après illustrent l'invention sans toutefois en limiter le domaine ou l'esprit.

Dans les exemples 1 à 35 ainsi que dans les essais témoins (a) à (f) le mode opératoire utilisé est le suivant:

Dans un autoclave dont la nature et la capacité seront précisées ci-après, on charge de l'acétate de méthyle, un ou plusieurs solvants, le cas échéant, et les divers constituants du système catalytique. Après fermeture de l'autoclave on admet une pression de monoxyde de carbone et une pression d'hydrogène, respectivement désignées par P (CO) et P ($H_2$) (valeurs mesurées à 25°C).

L'agitation, par un système de va-et-vient, est mise en route et l'autoclave est alors porté à la température choisie et designée par T, en 25 minutes environ. La pression totale en température, désignée par P(T), est maintenue sensiblement à la valeur indiquée par des recharges successives de monoxyde de carbone renfermant au maximum 1% (en volume) d'hydrogène. Après une durée de réaction désignée par t, on refroidit l'autoclave et on le dégaze. Le mélange réactionnel est alors analysé par chromatographie et potentiométrie.

Les autoclaves utilisés, désignés dans ce qui suit par A, B et C ont respectivement les caractéristiques suivantes:

- A: autoclave en tantale de 125 millilitres de capacité,
- B: autoclave en acier inoxydable Z-8 CNDT 17-12 (norme AFNOR) de 250 millilitres de capacité,
- C: autoclave en HASTELLOY B2 de 125 millilitres de capacité.

Dans les exemples les conventions suivantes sont utilisées:

- AcOMe: désigne l'acétate de méthyle
- AcOH: désigne l'acide acétique
- $Ac_2O$: désigne l'anhydride acétique
- TMS: désigne la tétraméthylènesulfone
- AcH: désigne l'acétaldehyde
- DAE: désigne le diacétate d'éthylidène
- Pr: désigne la productivité exprimé en gramme du produit considéré par heure et par litre
- meq: signifie milliéquivalent
- mAt-g: signifie milliatome-gramme
- mMol: signifie millimole.

Toutes les pressions [P(CO), P($H_2$) et P(T)] sont exprimées en bars.

*Exemples 1 à 9 - Essais temoins (a) à (d):*

Dans le tableau (I) ci-après on a rassemblé les conditions particulières ainsi que les résultats obtenus lors d'une première série d'essais réalisée avec du dicobaltoctacarbonyle comme source de cobalt, du triruthéniumdodécocarbonyle comme source de ruthénium, de l'iodure de méthyltriphénylphosphonium comme iodure ionique; dans l'autoclave A on a chargé 35 millilitres (ml) d'acétate de méthyle et 10 ml d'acide acétique; dans l'autoclave B on a chargé 70 ml d'acétate de méthyle et 20 ml d'acide acétique.

Les essais témoins (a), (c) et (d) montrent que le cobalt seul ne développe qu'une médiocre activité carbonylante. L'essai témoin (b) montre que le ruthénium seul ne développe pratiquement aucune activité carbonylante.

L'exemple 1, notamment, démontre l'efficacité remarquable du présent procédé.

*Exemples 10 à 16 - Essai temoin (e):*

Dans le tableau (II) ci-après on a rassemblé les conditions particulières ainsi que les résultats obtenus lors d'une seconde série d'essais réalisée dans l'autoclave C sur une charge constituée de:

- AcOMe: 25 ml
- AcOH: 5 ml
- TMS: 15 ml
- 1 mAt-g de cobalt sous forme de $Co_2(CO)_8$ (concentration en cobalt: 20 mAt-g/l)
- 1 mAt-g de ruthénium sous forme de $Ru_3(CO)_{12}$ (Ru/Co = 1)
- 15 mMol d'un iodure ionique dont la nature est précisée dans le tableau (II) ($I^-$/Co = 15)
- le cas échéant d'un acétate alcalin dont la nature et la quantité sont précisé dans le tableau (II).

Les conditions opératoires communes sont les suivantes:

- P(CO): = 80 bars
- $P(H_2)$ = 10 bars
- T = 180°C
- P(T) = 150 bars
- t = 120 minutes.

Lors de certains essais on a dosé en fin d'essai la quantité d'iodure de méthyle présente dans le milieu réactionnel. Cette quantité exprimée en millimoles est indiquée dans la colonne du tableau (II) intitulé $(CH_3I)$.

L'essai témoin (e) est réalisé en remplaçant dans la charge l'iodure ionique par 14 mMol d'iodure de méthyle.

L'essai témoin (e) démontre le caractère néfaste de l'iodure de méthyle dans le présent procédé.

Les exemples (11) et (13) montrent l'avantage obtenu par l'introduction dans le milieu réactionnel d'un carboxylate lorsque l'on fait appel aux iodures alcalins à titre d'iodure ionique.

*Exemples 17 à 28 - Essais temoins (f) et (g):*

Dans le tableau (III) ci-après on a rassemblé les conditions particulières ainsi que les résultats obtenus lors d'une troisième série d'essais réalisée dans l'autoclave B, en utilisant le diacétate de cobalt tétrahydraté comme source de cobalt (la concentration en cobalt est de 4,5 mAt-g/l), l'iodure de méthyltriphényl-phosphonium comme iodure ionique (l'exemple 23 étant toutefois réalisé en présence d'iodure de tétraéthylammonium), le triruthéniumdodécacarbonyle comme source de ruthénium (toutefois, dans l'exemple 18 on a fait appel à l'acétylacétonate de ruthénium), l'acide acétique étant le solvant utilisé. Toutefois dans les exemples 20 et 21 on ajoute à la charge respectivement 20 et 35 ml d'anhydride acétique; dans l'essai témoin (g) on a ajouté à la charge 15 mMol d'iodure de méthyle.

La température de réaction est de 210°C (sauf dans l'exemple 26 où elle est de 197°C); P(T) est de 250 bars (sauf dans l'exemple 22 où elle est de 150 bars seulement). La durée de réaction en température est de 20 mn (sauf dans les exemples 19 et 20 où elle est respectivement de 10 et 25 minutes).

Dans l'exemple 28 on a ajouté à la charge de 5 mMol de triphénylphosphine $[X_T/(m.A^{m+} + n.A'^{n+}) = 0,81]$.

Dans l'essai témoin (g) le rapport $X_T/(m.A^{m+} + n.A'^{n+})$ est égal à : 1,67.

*Exemples 29 à 34*

Dans le tableau IV ci-après on a rassemblé les conditions particulières ainsi que les résultats obtenus lors d'une quatrième série d'essais réalisée sur une charge comprenant:

- 20 ml de N-méthylpyrrolidone,
- 30 ml d'acétate de méthyle
- 1,5 mAt-g de cobalt
- du ruthénium (2 mAt-g dans les exemples 29 à 33, Ru/Co = 1,33; 0,2 mAt-g dans l'exemple 34, Ru/Co = 0,133)
- de l'iodure de lithium.

Dans les exemples 30 et 32, on charge également 3 mMol d'acétate de lithium. Dans l'exemple 33 on charge également 5 mMol d'acétate de magnésium.

Les conditions particulières ainsi que les résultats obtenus figurent dans le tableau IV ci-après dans lequel:

- $Co(OAc)_2$ désigne l'acétate de cobalt tétrahydraté
- $Ru(acac)_3$ désigne l'acétylacétonate de ruthénium
- Ru/C désigne du ruthénium déposé sur charbon (la masse totale de ce catalyseur est de 400 mg).

*Exemple 35*

Dans un autoclave en tantale on réalise un essai sur une charge constituée par:

- 30 ml d'acétate de méthyle
- 20 ml de N-méthylpyrrolidone
- 4,5 mAt-g de cobalt sous forme d'acétate tétrahydraté
- 2 mAt-g de ruthénium sous forme de triruthéniumdodécacarbonyle (Ru/Co = 0,44)
- 9,6 mMol d'iodure de lanthane ($I^-$ = 28,8 meq).

Les conditions opératoires sont les suivantes:

- P (CO) = 36; p $(H_2)$ = 7
- T = 130°C
- P (T) = 60
- t = 155 mn.

En fin d'essai, on détecte par chromatographie en phase gazeuse la présence de 3,6 g d'anhydride acétique.

*Exemple 36*

Dans un réacteur en tantale de 125 ml de capacité on introduit:

- 20 ml de N-méthylpyrrolidone
- 30 ml d'acétate de méthyle
- 1,5 mAt-g de cobalt sous forme d'acétate tétrahydraté
- 2 mAt-g de ruthénium sous forme de triruthéniumdodécacarbonyle
- 20 mMol d'acétate de calcium.
- 40 mMol d'iodure de méthyle.

Après fermeture de l'autoclave on admet du monoxyde de carbone (36 bars à 25°C) et de l'hydrogène (7 bars à 25°C). L'agitation par un système de va-et-vient est mise en route et l'autoclave est porté à 70°C en 10 minutes environ. La pression totale à cette température atteint 50 bars, et elle est maintenue à cette valeur par des recharges successives de monoxyde de carbone renfermant au maximum 1% (en volume) d'hydrogène. Après 190 minutes à cette température, on porte l'autoclave à 120°C. La pression totale à cette température est de 60 bars; elle est maintenue à cette valeur comme indiqué ci-avant. Après 160 minutes à cette température, on refroidit l'autoclave et on le dégaze. Le mélange réactionnel est alors analysé par chromatographie en phase gazeuse. Il contient 6,5 g d'anhydride acétique.

*Exemples 37 à 40 - Essai temoin (h):*

Dans les exemples 37 à 40 ainsi que dans l'essai témoin (h) le mode opératoire est le suivant:

Dans un autoclave de 300 $cm^3$ en acier inoxydable muni d'une agitation centrale à entraînement magnétique, chauffé et régulé électriquement, on introduit:

- Ac OMe: 77 g
- Ac OH: 17,4 g
- N-méthyylpyrrolidone: 51,3 g
- Iodure de méthyltriphénylphosphonium: 50 mMol
- 1,64 mAt-g de cobalt sous la forme de diacétate tétrahydraté
- 1,64 mAt-g de ruthénium sous la forme d'acétylacétonate

puis on chauffe, sous balayage de monoxyde de carbone et d'hydrogène, jusqu'à une température de 200°C. La pression dans l'autoclave est maintenue à 235 bars et le débit d'alimentation du mélange gazeux est de 40 l/h (CNTP). Le pourcentage molaire d'hydrogène dans ce débit d'alimentation est maintenu constant et est indiqué dans le tableau ('') ci-après.

On effectue périodiquement des prélèvements de la masse réactionnelle que l'on analyse.

Dans le tableau (V) ci-après on a indiqué le pourcentage d'hydrogène dans l'alimentation par % $H_2$; TT Ac OMe est le pourcentage molaire d'acétate de méthyle transformé par rapport à la quantité chargée, respectivement au bout de 1 heure et de 4 heures de fonctionnement, RT ($Ac_2O$) est la quantité (exprimé en pourcents molaires) d'anhydride acétique formée par rapport à la quantité d'acétate de méthyle transformée, au bout de quatre heures de fonctionnement. RT (HYD) est la quantité (exprimée en pourcents molaires) de produits d'hydrocarbonylation (AcH et DAE) formée par rapport à la quantité d'acétate de méthyle transformée, au bout de 4 heures de fonctionnement.

TABLEAU I

| Ex n° | (*) | Cobalt mAt-g | Cobalt mAt-g/l | Ru mAt-g | Ru/Co | $I^-$ meq | $I^-$/Co | P(CO) | P($H_2$) | T (°C) | P(T) | t (mn) | $Ac_2O$ (g) | $Ac_2O$ Pr | DAE (g) | AcH (g) |
|---|---|---|---|---|---|---|---|---|---|---|---|---|---|---|---|---|
| a | A | 1 | 20 | 0 | 0 | 15 | 15 | 130 | 10 | 210 | 250 | 36 | 1,61 | 54 | 0 | 0,08 |
| b | » | 0 | 0 | 1 | — | » | | » | » | » | » | 35 | 0,39 | 13 | 0 | 0,23 |
| 1 | » | 1 | 20 | 0,5 | 0,5 | » | 15 | » | » | » | » | 20 | 8,37 | 500 | 0,25 | 0,19 |
| 2 | B | 2 | » | 1 | » | 30 | » | » | » | » | » | » | 17,86 | 535 | 0,57 | 0,54 |
| 3 | » | » | » | 0,04 | 0,02 | » | » | » | » | » | » | 50 | 14,28 | 170 | 1,07 | 0,69 |
| 4 | » | » | » | 0,2 | 0,1 | » | » | » | » | » | » | 33 | 14,90 | 270 | 0,62 | 0,61 |
| 5 | » | » | » | 2 | 1 | » | » | » | » | » | » | 20 | 19,99 | 600 | 0,69 | 0,42 |
| 6 | » | 0,45 | 20 | 1,3 | 2,89 | 15 | 33 | » | » | » | » | » | 25,40 | 760 | 0,47 | 0,67 |
| c | A | 2 | 40 | 0 | 0 | 22,5 | 11,2 | 54 | 4 | 180 | 100 | » | 1,1 | 45 | ND | ND |
| 7 | » | » | » | 1 | 0,5 | » | » | » | » | » | » | » | 3,48 | 140 | » | » |
| d | » | » | » | 0 | 0 | » | » | 39 | 3 | 210 | » | » | 0,58 | 23 | » | » |
| 8 | » | » | » | 1 | 0,5 | » | » | » | » | » | » | » | 2,97 | 120 | » | » |
| 9 | » | » | » | 1 | » | » | » | 135 | 10 | 180 | 250 | » | 6,06 | 240 | » | » |

(*): type d'autoclave utilisé - ND: non déterminé.

TABLEAU II

| Ex n° | $A^{m+}\ I^{-}_{m}$ | $A'^{n+}\ (OCOCH_3)^{-}_{n}$ nature | mMol | $X_T/(m.A^{m+} + n.A'^{n+})$ | $Ac_2O$ (g) | $(CH_3I)$ |
|---|---|---|---|---|---|---|
| e | néant | — | 0 | — | 0,3 | 10 |
| 10 | $Li^+I^-$ | — | 0 | 1 | 2,25 | 4,4 |
| 11 | » | $Li^+(OAc)^-$ | 15 | 0,5 | 3,57 | 3,5 |
| 12 | $Na^+I^-$ | — | 0 | 1 | 4,05 | 0,9 |
| 13 | » | $Na^+(OAc)^-$ | 15 | 0,5 | 5,3 | 0,2 |
| 14 | » | » | 60 | 0,2 | 3,57 | » |
| 15 | $K^+I^-$ | » | 0 | 1 | 1,78 | ND |
| 16 | $P^+I^-$ (*) | — | 0 | 1 | 8,3 | 0,2 |

(*): $CH_3(C_6H_5)_3P^+I^-$

TABLEAU III

| Ex n° | AcOMe ml | AcOH ml | Cobalt mAt-g | Ru/Co | $I^-$ meq | $I^-/Co$ | P(CO) | $P(CH_2)$ | $Ac_2O$ g | Pr | DAE g | Pr | AcH (g) |
|---|---|---|---|---|---|---|---|---|---|---|---|---|---|
| 17 | 70 | 20 | 0,45 | 2,88 | 45 | 100 | 70 | 70 | 11,80 | 350 | 6,33 | 190 | 2,80 |
| 18 | » | » | » | » | » | » | » | » | 11,23 | 335 | 5,52 | 165 | 4,35 |
| 19(*) | » | » | » | » | » | » | » | » | 3,2 | 190 | 1,66 | 100 | 6,2 |
| 20 | 50 | » | » | » | » | » | 105 | » | 21,07 | 0 | 14,78 | 355 | 2,5 |
| 21 | 35 | » | » | » | » | » | 120 | 20 | 46,19 | 275 | 2,96 | 90 | 0,5 |
| 22 | 70 | 20 | » | » | » | » | 70 | 10 | 10,96 | 330 | ND | ND | ND |
| 23 | » | » | » | » | » | » | 125 | 15 | 16,32 | 490 | ND | » | ND |
| 24 | » | » | » | 0,75 | » | » | 130 | 10 | 10,04 | 300 | » | » | » |
| f | » | » | » | » | » | » | 140 | 0 | 3,0 | 90 | » | » | » |
| 25 | 45 | 0 | 0,23 | 2,87 | 22,5 | 98 | 125 | 15 | 1,94 | 115 | » | » | » |
| 26 | 35 | 10 | » | » | » | » | 70 | 70 | 4,9 | 290 | » | » | » |
| 27 | » | » | » | » | 15 | 65 | 120 | 20 | 10,40 | 620 | » | » | » |
| 28 | » | » | » | » | 22,5 | 98 | » | » | 11,12 | 670 | » | » | » |
| g | » | » | » | » | » | » | 125 | 15 | 2,04 | 120 | » | » | » |

(*): dans cet essai P(T) a été maintenue constante par des recharges d'un mélange $CO/H_2$ : 1/1 (molaire)
ND: non déterminé

TABLEAU IV

| Ex n° | (*) | Cobalt nature | Ruthénium nature | $I^-$ meq | P(CO) | $P(H_2)$ | T °C | P(T) | t mn | $Ac_2O$ g | DAE g |
|---|---|---|---|---|---|---|---|---|---|---|---|
| 29 | A | $Co_2(CO)_8$ | $Ru_3(CO)_2$ | 30 | 32 | 7 | 154 | 60 | 456 | 27 | 0,3 |
| 30 | C | $CoI_2$ | $Ru(acac)_3$ | 67 | 36 | 7 | 125 | 60 | 275 | 6 | ND |
| 31 | A | $Co(OAc)_2$ | $Ru_3(CO)_{12}$ | 70 | 36 | 7 | 120 | 60 | 340 | 22 | 0,15 |
| 32 | A | $CoI_2$ | $Ru_3(CO)_{12}$ | 60 | 40 | 8 | 95 | 60 | 155 | 1,3 | ND |
| 33 | A | $Co(OAc)_2$ | $Ru_3(CO)_{12}$ | 40 | 19 | 3 | 120 | 30 | 405 | 10,7 | ND |
| 34 | A | $Co_2(CO)_8$ | Ru/C | 30 | 32 | 7 | 154 | 60 | 375 | 18,5 | 0,17 |

(*): Type d'autoclave utilisé - ND: non determiné

TABLEAU V

| Ex n° | % $H_2$ | TT Ac OMe 1 h | 4 h | RT ($AC_2O$) | RT (HYD) |
|---|---|---|---|---|---|
| h | 0 | 16 | 35 | 100 | 0 |
| 37 | 1 | 31 | 78 | 100 | 0 |
| 38 | 2 | 51 | 95 | 99,4 | 0,6 |
| 39 | 5 | 59 | 96 | 94 | 6 |
| 40 | 10 | 59 | 97 | 84 | 16 |

**Revendications**

1. Procédé de préparation de l'anhydride acétique le cas échéant en mélange avec l'acétaldéhyde, et/ou le diacétate d'éthylidène par réaction en phase liquide homogène dans un milieu pratiquement anhydre de l'acétate de méthyle et du monoxyde de carbone, caractérisé en ce qu'on opère en présence simultanée:

— (a) d'une source de cobalt,
— (b) d'une source de ruthénium,
— (c) d'un iodure ionique de formule:

$$A^{m+}\ I^-_m$$

dans laquelle $A^{m+}$ est un cation choisi dans le groupe constitué par les cations onium quaternaire dérivant des éléments du groupe de l'azote et les cations des métaux alcalins, les cations des métaux alcalino-terreux, les cations des métaux du groupe des lanthanides et les cations des métaux du groupe des actinides, m étant un entier égal à 1, 2, 3 ou 4,

— (d) le cas échéant, d'un carboxylate de formule:

$$A'^{n+}\ (OCOR)^-_n$$

dans laquelle n est égal à 1, 2, 3 ou 4 et $A'^{n+}$ a la signification donnée ci-avant pour $A^{m+}$, $A'^{n+}$ et $A^{m+}$ pouvant par ailleurs être identiques ou différents, et R est un radical alkyle, aralkyle ou aryle ayant au maximum 8 atomes de carbone, et

—(e) d'hydrogène, l'hydrogène représentant de 1 à 50% en volume du mélange de monoxyde de carbone et d'hydrogène, la température de réaction étant comprise entre 60 et 300°C, et la pression totale en température étant comprise entre 20 et 300 bars, et la quantité totale de composés halogénés présente dans le milieu réactionnel (exprimée en atome-gramme d'halogène et désignée par $X_T$) étant telle que le rapport atomique $X_T/(m.A^{m+}+n.A'^{n+})$ soit inférieur ou égal à 1.

2. Procédé selon la revendication 1, caractérisé en ce que la température est comprise entre 80 et 240°C.

3. Procédé selon la revendication 1 ou 2, caractérisé en ce que la concentration en cobalt est comprise entre 0,1 et 200 mAt-g/l.

4. Procédé selon l'une quelconque des revendications précédentes, caractérisé en ce que le rapport molaire $I^-/Co$ est supérieur ou égal à 5 et, de préférence supérieur ou égal à 10.

5. Procédé selon l'une quelconque des revendications précédentes, caractérisé en ce que le rapport molaire $I^-/Co$ est compris entre 15 et 150.

6. Procédé selon l'une quelconque des revendications précédentes, caractérisé en ce que le rapport atomique Ru/Co est compris entre 0,005 et 25 et, de préférence entre 0,02 et 10.

7. Procédé selon l'une quelconque des revendications précédentes, caractérisé en ce que le rapport atomique Ru/Co est compris entre 0,2 et 5.

8. Procédé selon l'une quelconque des revendications précédentes, caractérisé en ce que l'iodure ionique est un iodure de phosphonium quaternaire ou d'ammonium quaternaire.

9. Procédé selon l'une quelconque des revendications 1 à 7, caractérisé en ce que l'iodure ionique est un iodure alcalin, un iodure alcalino-terreux, un iodure d'un métal du groupe des lanthanides ou un iodure d'un métal du groupe des actinides.

10. Procédé selon la revendication 9, caractérisé en ce que l'iodure ionique est un iodure alcalin.

11. Procédé selon la revendication 10, caractérisé en ce que l'iodure ionique est l'iodure de lithium.

12. Procédé selon l'une quelconque des revendications 9 à 11, caractérisé en ce que l'on opère en présence d'un carboxylate de formule:

$$A'^{n+}\ (OCOR)^-_n$$

dans laquelle n, $A'^{n+}$ et R ont la signification donnée dans la revendication 1, le rapport molaire $A'^{n+}/A^{m+}$ étant compris entre 0,01 et 20.

13. Procédé selon l'une quelconque des revendications 9 à 12, caractérisé en ce que la réaction est conduite à une température inférieure à 160°C, sous une pression totale en température inférieure à 100 bars et dans un solvant choisi parmi la tétraméthylènesulfone, la N-méthylpyrrolidone et les amides d'acides monocarboxyliques qui dérivent d'acides ayant au maximum 8 atomes de carbone, et dont l'atome d'azote comporte deux substituants alkyles ayant au maximum 4 atomes de carbone.

14. Procédé selon l'une quelconque des revendications 1 à 12, caractérisé en ce que la réaction est conduite à une température supérieure ou égale à 160°C et sous une pression totale en température supérieure ou égale à 100 bars.

15. Procédé selon la revendication 14, caractérisé en ce que le milieu réactionnel renferme un solvant choisi parmi les acides carboxyliques aliphatiques ayant au maximum 8 atomes de carbone.

16. Procédé selon la revendication 15, caractérisé en ce que le solvant est l'acide acétique.

**Patentansprüche**

1. Verfahren zur Herstellung von Essigsäureanhydrid, gegebenenfalls im Gemisch mit Acetaldehyd und/oder Ethylidendiacetat, durch Umsetzung eines Gemisches aus Methylacetat und Kohlenmonoxid in flüssiger Phase in einem praktisch wasserfreien Medium, dadurch gekennzeichnet, dass man in gleichzeitiger Anwesenheit von:

— (a) einer Kobaltquelle,
— (b) einer Rutheniumquelle,
— (c) eines ionischen lodids der Formel

$$A^{m+}\ I^{-}m,$$

in der $A^{m+}$ ein Kation ist, ausgewählt aus der Gruppe bestehend aus den quaternären Oniumkationen, abgeleitet von den Elementen der Stickstoff-Gruppe, den Alkalikationen, den Kationen der Erdalkalimetalle, den Kationen der Metalle der Gruppe der Lanthaniden und den Kationen der Metalle der Gruppe der Actinoiden, m eine ganze Zahl mit dem Wert 1, 2, 3 oder 4 ist, und

— (d) gegebenenfalls einem Carboxylat der Formel

$$A'^{n+}\ (OCOR)^{-}_{n}$$

in der n = 1, 2, 3 oder 4 und $A'^{n+}$ die oben für $A^{m+}$ angegebene Bedeutung hat, wobei $A'^{n+}$ und $A^{m+}$ gleich oder verschieden sein können, und R eine Alkyl-, Aralkyl- oder Arylgruppe mit maximal 8 Kohlenstoffatomen ist, und

— (e) Wasserstoff arbeitet, der Wasserstoff 1 bis 50 Vol.-% des Gemisches aus Kohlenmonoxid und Wasserstoff ausmacht, die Reaktionstemperatur 60 bis 300°C beträgt, der Gesamtdruck bei der angewandten Temperatur 20 bis 300 bar ausmacht und die Gesamtmenge der im Reaktionsmedium vorhandenen Halogenverbindungen (angegeben in g-Atom Halogen und bezeichnet mit $X_T$) so ist, dass das Atomverhältnis $X_T/(m.A^{m+} + n.A'^{n+})$ weniger als 1 oder gleich 1 ist.

2. Verfahren nach Anspruch 1, dadurch gekennzeichnet, dass die Temperatur 80 bis 240°C beträgt.

3. Verfahren nach Anspruch 1 oder 2, dadurch gekennzeichnet, dass die Cobaltkonzentration 0,1 bis 200 mgAtom/l beträgt.

4. Verfahren nach einem der vorangehenden Ansprüche, dadurch gekennzeichnet, dass das Molverhältnis $I^{-}/Co \geqq 5$ und vorzugsweise $\geqq 10$ ist.

5. Verfahren nach einem der vorangehenden Ansprüche, dadurch gekennzeichnet, dass das Molverhältnis $I^{-}/Co$ 15 bis 150 beträgt.

6. Verfahren nach einem der vorangehenden Ansprüche, dadurch gekennzeichnet, dass das Atomverhältnis Ru/Co 0,005 bis 25 und vorzugsweise 0,02 bis 10 ausmacht.

7. Verfahren nach einem der vorangehenden Ansprüche, dadurch gekennzeichnet, dass das Atomverhältnis Ru/Co 0,2 bis 5 ausmacht.

8. Verfahren nach einem der vorangehenden Ansprüche, dadurch gekennzeichnet, dass das ionische lodid ein quaternäres Phosphoniumiodid oder ein quaternäres Ammoniumchlorid ist.

9. Verfahren nach einem der Ansprüche 1 bis 7, dadurch gekennzeichnet, dass das ionische lodid ein Alkaliiodid, ein Erdalkaliiodid, ein lodid eines Metalls der Gruppe der Lanthaniden oder ein lodid eines Metalls der Gruppe der Actinoiden ist.

10. Verfahren nach Anspruch 9, dadurch gekennzeichnet, dass das ionische lodid ein Alkaliiodid ist.

11. Verfahren nach Anspruch 10, dadurch gekennzeichnet, dass das ionische lodid ein Lithiumiodid ist.

12. Verfahren nach einem der Ansprüche 9 bis 11, dadurch gekennzeichnet, dass man in Anwesenheit eines Carboxylats der Formel

$$A'^{n+}\ (OCOR)^{-}_{n}$$

arbeitet, in der n, $A'^{n+}$ und R die in Anspruch 1 angegebene Bedeutung haben und das Molverhältnis $A'^{n+}/A^{m+}$ 0,01 bis 20 beträgt.

13. Verfahren nach einem der Ansprüche 9 bis 12, dadurch gekennzeichnet, dass die Umsetzung bei einer Temperatur unterhalb 160°C, unter einem Gesamtdruck bei der angewandten Temperatur unterhalb 100 bar und in einem Lösungsmittel, ausgewählt unter Tetramethylensulfon, N-Methylpyrrolidon und Monocarbonsäureamiden, abgeleitet von Säuren mit maximal 8 Kohlenstoffatomen, deren Stickstoffatom 2 Alkylsubstituenten mit maximal 4 Kohlenstoffatomen trägt, durchgeführt wird.

14. Verfahren nach einem der Ansprüche 1 bis 12, dadurch gekennzeichnet, dass die Umsetzung bei einer Temperatur über oder gleich 160°C und unter einem Gesamtdruck bei der angewandten Temperatur von über oder gleich 100 bar durchgeführt wird.

15. Verfahren nach Anspruch 14, dadurch gekennzeichnet, dass das Reaktionsmedium ein Lösungsmittel, ausgewählt aus den aliphatischen Carbonsäuren mit maximal 8 Kohlenstoffatomen, umfasst.

16. Verfahren nach Anspruch 15, dadurch gekennzeichnet, dass das Lösungsmittel Essigsäure ist.

**Claims**

1. Process for the preparation of acetic anhydride mixed, should the occasion arise, with acetaldehyde and/or ethylidene diacetate, by reaction, in homogeneous liquid phase in an almost anhydrous medium, of methyl acetate and carbon monoxide, characterised in that the reaction is performed in the simultaneous presence of:

— (a) a source of cobalt,
— (b) a source of ruthenium,
— (c) an ionic iodide of the formula:

$$A^{m+}\ I^{-}m$$

in which $A^{m+}$ is a cation chosen from the group consisting of the quaternary onium cations derived from nitrogen group elements and the cations of alkali metals, of alkaline earth metals, of lanthanide group metals and of actinide group metals, m being an integer equal to 1, 2, 3 or 4,

— (d) should the occasion arise, a carboxylate of the formula

$$A'^{-n+}\ (OCOR)^{-}_{n}$$

in which n equals 1, 2, ,3 or 4 and $A'^{-n+}$ has the significance given above for $A^{M+}$, where $A'^{n+}$ and $A^{m+}$ can moreover be identical or different, and R is an alkyl, aralkyl or aryl radical having a maximum of 8 carbon atoms, and

— (e) hydrogen, the hydrogen representing from 1 to 50% by volume of the mixture of carbon monoxide and hydrogen, the reaction temperature being between 60 and 300°C, the total pressure being between 20 and 300 bars at the working temperature, and the total quantity of halogen compounds present in the reaction medium (expressed in gram-atoms of halogen and designated by $X_T$) being such that the atomic ratio $X_T/(mA^{m+} + nA'^{n+})$ is less than or equal to 1.

2. Process according to claim 1, characterised in that the temperature is between 80 and 240°C.

3. Process according to claim 1 or 2, characterised in that the cobalt concentration is between 0.1 and 200 mg-At/l.

4. Process according to any one of the preceding claims, characterised in that the mole ratio $I^-/Co$ is greater than or equal to 5, and preferably greater than or equal to 10.

5. Process according to any one of the preceding claims, characterised in that the mole ratio $I^-/Co$ is between 15 and 150.

6. Process according to any one of the preceding claims, characterised in that the atomic ratio Ru/Co is between 0.005 and 25, and preferably between 0.02 and 10.

7. Process according to any one of the preceding claims, characterised in that the atomic ratio Ru/Co is between 0.2 and 5.

8. Process according to any one of the preceding claims, characterised in that the ionic iodide is a quaternary phosphonium iodide or quaternary ammonium iodide.

9. Process according to any one of claims 1 to 7, characterised in that the ionic iodide is an alkali metal iodide, alkaline earth metal iodide, lanthanide group metal iodide or actinide group metal iodide.

10. Process according to claim 9, characterised in that the ionic iodide is an alkali metal iodide.

11. Process according to claim 10, characterised in that the ionic iodide is lithium iodide.

12. Process according to any one of claims 9 to 11, characterised in that the reaction is performed in the presence of a carboxylate of formula:

$$A'^{n+} (OCOR)^-_n$$

in which n, $A'^{n+}$ and R have the significance given in claim 1, the mole ratio $A'^{n+}/A^{m+}$ being between 0.01 and 20.

13. Process according to any one of claims 9 to 12, characterised in that the reaction is performed at a temperature below 160°C, at a total pressure of less than 100 bars at the working temperature and in a solvent chosen from tetramethylenesulphone, N-methylpyrrolidone and amides of monocarboxylic acids derived from acids having a maximum of 8 carbon atoms and in which the nitrogen atom bears two alkyl substituents having a maximum of 4 carbon atoms.

14. Process according to any one of claims 1 to 12, characterised in that the reaction is performed at a temperature above or equal to 160°C and at a total pressure greater than or equal to 100 bars at the working temperature.

15. Process according to claim 14, characterised in that the reaction medium contains a solvent chosen from the aliphatic carboxylic acids having a maximum of 8 carbon atoms.

16. Process according to claim 15, characterised in that the solvent is acetic acid.